# EUROPEAN PATENT SPECIFICATION

(11) **EP 2 303 381 B1**
(45) Date of publication and mention of the grant of the patent: **14.10.2020**
(21) Application number: 09773828.0
(22) Date of filing: 01.07.2009
(51) Int. Cl.: A61B 8/00, A61B 1/00, A61B 46/17

(54) **ULTRASOUND PROBE COVER AND METHOD FOR ITS PRODUCTION**
SCHUTZHÜLLE FÜR EINE ULTRASCHALLSONDE UND HERSTELLUNGSVERFAHREN DAFÜR
CAPUCHON DE SONDE À ULTRASON ET SON PROCÉDÉ DE FABRICATION

(30) Priority: 03.07.2008 US 16736008
(43) Date of publication of application: 06.04.2011
(73) Proprietor: Ascendia AB, 182 33 Danderyd (SE)
(72) Inventor: ÅHLUND, Patrick, S-187 68 Täby (SE); LINDGREN, Mikael, S-194 77 Upplands Väsby (SE)
(74) Representative: Schneiders, Josef
(86) International application number: PCT/SE2009/000345
(87) International publication number: WO 2010/002313

(56) References cited:
- EP-A1- 0 477 581
- EP-A2- 0 104 618
- WO-A1-97/42475
- WO-A1-2006/023352
- WO-A1-2008/127886
- GB-A- 2 396 562
- US-A- 4 887 615
- US-A- 5 676 159
- US-A- 5 997 481
- US-A- 6 051 293
- US-A1- 2003 093 086
- US-A1- 2005 049 460
- US-B1- 6 416 462

## Description

### FIELD OF THE INVENTION

The present invention relates to an ultrasound probe cover and a method for its manufacture.

### BACKGROUND OF THE INVENTION

Ultrasound probes cannot be easily sterilized. To protect the patient from microbial contamination ultrasound probes used in diagnosis therefore are inserted into a thin sheath of polymer material. Since air is a bad ultrasound conductor, air bubbles between the probe and the sheath have to be avoided. This is accomplished by filling the lumen of the probe partially with an aqueous ultrasound conducting gel, which provides ultrasound conducting contact between the probe and the inner face of the sheath. It is also clinically necessary to provide such gel to the outer face of the sheath, although this aspect is of no interest to the present invention.

A great number of ultrasound probe covers are known in the art. They comprise or consist of a tubular sheath of plastic material closed at its one, front end, or of dipped covers made from latex, neoprene, chloroprene and other resilient polymers. Known tubular sheaths of polymer material usually have welded seams along their entire length, which increases their risk for leakage. Prior to use the ultrasound probe is inserted through the open rear end of the sheath and displaced in the direction of the front end until abutment with the inner face of the latter. While some ultrasound probe covers are marketed partially filled with ultrasound conducting gel at their front end portion other probe covers are filled with gel prior to use.

Ultrasound probe covers known in the art are made from thin polymer foil. The foil is cut to appropriate shape and heat sealed at its edges to form a tube with a closed end and an open end. A problem with ultrasound probes of this kind is their bad fit with ultrasound probe heads. Another, concomitant problem is the existence of ears and other kind of more or less sharp protrusions of polymer material at the closed end portion of the cover, which ears or other protrusions are irritating and may even damage sensitive mucosa in the body cavities into which the ultrasound probe provided with the cover is inserted. The protruding portions of the cover are at risk to be penetrated by a biopsy needle disposed in close proximity of the probe head, which would destroy the sterile barrier formed by the cover. Protruding portions of the sheath may also hinder the mounting of a biopsy needle guide on the probe.

While ultrasound probe covers made by a dipping process do not have these drawbacks, they are generally more expensive, difficult to make in larger size, and sometimes allergenic, e.g., if made of latex.

US 4 887 615 discloses an ultrasound probe cover comprising a flexible polymer tube member and a top member joined to each other by welding or by use of an adhesive.

EP 0 477 581 discloses an ultrasound probe cover consisting of a plastic foil tube wherein one end is closed by welding and wherein the weld seam lies on the inside.

EP 0 104 618 A2 **describes a probe cover that is made of a puncture-proof cap and a flexible tubular extension, but there is no mentioning of a process of manufacturing said probe cover and protruding seams seem to be unavoidable.**

### OBJECTS OF THE INVENTION

A first object of the invention is to provide an ultrasound probe cover that is well adapted to ultrasound probe heads used in the art.

Another object of the invention is to provide an ultrasound probe cover that does not irritate or damage sensitive mucosa in the body of a patient.

Still another object of the invention is to provide an ultrasound probe cover fitting well to the probe head and lacking protruding portions that risk to be penetrated by a biopsy needle disposed in close proximity of the probe head or which may complicate the mounting of a needle guide attachment.

Further objects of the invention will become evident from the following summary of the invention, the description of a number of preferred embodiments illustrated in a drawing, and the appended claims.

### SUMMARY OF THE INVENTION

According to the present invention an ultrasound probe cover is disclosed comprising a flexible polymer tube member having an open first end and a second end closed by a top member of preferably same material and wherein the originally separate tube and top members are joined to each other adhesively. "Adhesively" includes the integration of the tube and top members at the joint by heating the portions of the material to be joined in an abutting relationship under pressure to a welding temperature or by use of an adhesive. A preferred adhesive comprises a solution in a suitable organic solvent of a polymer of same kind as the polymer of the tube and top members. A preferred welding temperature is a temperature in the vicinity of the melting point of the polymer. It is preferred for the tube member of the invention to be made by cutting a tube produced by polymer extrusion to appropriate length. The tube member of the invention can be of cylindrical form. Alternatively the tube member of the invention can be of varying diameter, such as having the form of a frustrum of a cone or comprising a throat. A tube member of varying diameter can be made from a cylindrical tube member by, for instance, thermoforming. A tube member produced by polymer extrusion does not comprise a weld or seam. It is however within the ambit of the invention to make the tube member from a polymer tube comprising one or several heat welding or adhesive seams extending in a longitudinal direction of the tube, such as by joining opposite edges of a rectangular piece of polymer foil by heat welding or gluing, respectively. It is preferred for the top member to be of circular or elliptic form or of a rectangular form with rounded corners. It is particularly preferred for the ultrasound probe cover to substantially comprise a tube member and top member of this kind joined in the aforementioned manner. It is also preferred for the top member to not comprise a seam or other discontinuity.

According to a preferred aspect of the invention, the top member is substantially flat. According to another preferred aspect of the invention, the top member is not flat, for instance it is dome shaped, or comprises a central dome shaped portion. A dome shaped top member or other non-flat top members can be produced by thermoforming to make them fit to probe heads of particular form.

The probe cover of the invention may be made of any thermoplastic material that is medically acceptable and of which two pieces can be joined by heating them under pressure in an abutting configuration to a welding temperature. Preferred thermoplastic polymer materials for use in the invention include polyethylene, polypropylene, and polyurethane. Polyurethane and polymers comprising polyurethane domains are particularly preferred materials. The thickness of the material of the tube member wall and of the top member is from 4 µm to 200 µm, preferably from 20 µm to 100 µm. It is also preferred for the material of the tube member and of the top member to be of the same thickness. The joining of the tube member and the top member by heat welding produces a seam extending along the periphery of the tube member's second end and of the top member. The seam so produced initially extends outwardly of the probe cover in the form of a narrow flange. The final configuration of the probe cover is obtained by turning it inside out. The probe cover of the invention can be dimensioned so as to suit ultrasound probe heads of a desired size and form.

According to a further preferred aspect of the invention, the ultrasound probe cover comprises a suitable amount of ultrasound gel in its lumen at its second, distal end, optionally in a distal compartment separated from the main distal portion of the probe cover by a transverse wall. The transverse wall should be arranged easily penetrable by the probe head during the process of insertion of the latter into the probe cover. The transverse wall can be arranged by applying heat and pressure to the flattened tube member along a line perpendicular to the tube axis. A temporarily closed proximal compartment containing ultrasound gel may be also arranged by substituting the transverse wall by clamping the tube member by a clamp or a similar device in a direction transverse to the tube axis distally of the ultrasound gel or by folding the probe cover in a transverse direction distally of the ultrasound gel and temporarily securing the fold from outside.

Other features and advantages of the present invention will become apparent from the following description of the invention which refers to the accompanying drawings.

### BRIEF DESCRIPTION OF THE DRAWIINGS

The invention will now be explained in greater detail by reference to preferred embodiments and apparatus for their manufacture illustrated in a rough drawing, in which
- Fig. 1: is a prior art probe head cover, in a perspective view;
- Fig. 2: is a prior art cut out sheet of polymer foil from which the prior art probe head cover of Fig. 1 has been made;
- Fig. 3: is a first embodiment of the probe head cover of the invention, in a perspective view;
- Fig. 4: is a second embodiment of the probe head cover of the invention, in a perspective view;
- Fig. 5: is a third embodiment of the probe head cover of the invention, in a perspective view;
- Fig. 6: is a fourth embodiment of the probe head cover of the invention, in a perspective view;
- Fig. 7: is an partial axial sectional view of the probe head cover of Fig. 2, enlarged and prior to turning it inside out;
- Fig. 8: is the probe head cover of Fig. 6 turned inside out, in the same enlarged view;
- Fig. 9: is an apparatus for manufacture of the probe head cover of the invention having a flat top member;
- Fig. 10: is an apparatus for manufacture of the probe head cover of the invention having a domed top member;
- Fig. 11: is a further embodiment of the probe head cover of the invention, in a perspective view;
- Figs. 12 - 12c: are axial sections through exemplary non-cylindrical tube members of the invention made by thermoforming cylindrical tube blanks.

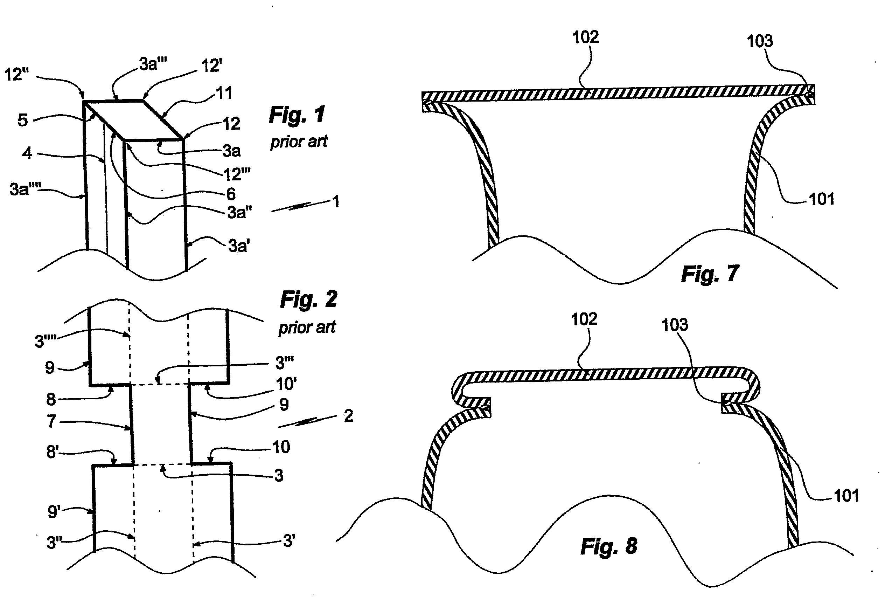

For clarity reasons, the probe head covers of Figs. 1 to 8 and 11 are shown in an inflated state by putting their lumen under a slight positive pressure. For the same reasons, in the figures representing sectional views, the wall thickness of polymer material is greatly exaggerated and dimensions are generally not to scale.

The prior art probe head cover 1 of Fig. 1 has been made by cutting a thin sheet 2 (Fig. 2) of polyurethane to size, plying the sheet 2 along plying lines 3, 3', 3", 3"', 3"" over a parallelepipedal mandrel (not shown) to form a probe head cover 1 of same form with corresponding edges 3a, 3a', 3a", 3a"', 3a"", and connecting cut edges 8, 8' with cut edge 7, cut edge 9 with cut edge 9', and cut edges 10, 10' with cut edge 9 by gluing or welding to form seams 5, 6, 4 and 11, respectively. The thus produced probe head cover 1 then is turned inside out. Its corners 12, 12', 12", 12"' are preserved as a kind of ears after turning the cover 1 inside out. When an ultrasound probe is clad with the prior art cover 1 the ears 12, 12', 12", 12'" stand out from the probe 1. They are prone to irritate sensitive mucosal tissue of a patient undergoing ultrasound diagnosis. Moreover, the ears 12, 12', 12", 12'" have a tendency to retain air. Air bubbles trapped in the cover 1 will reduce the quality of the ultrasound image.

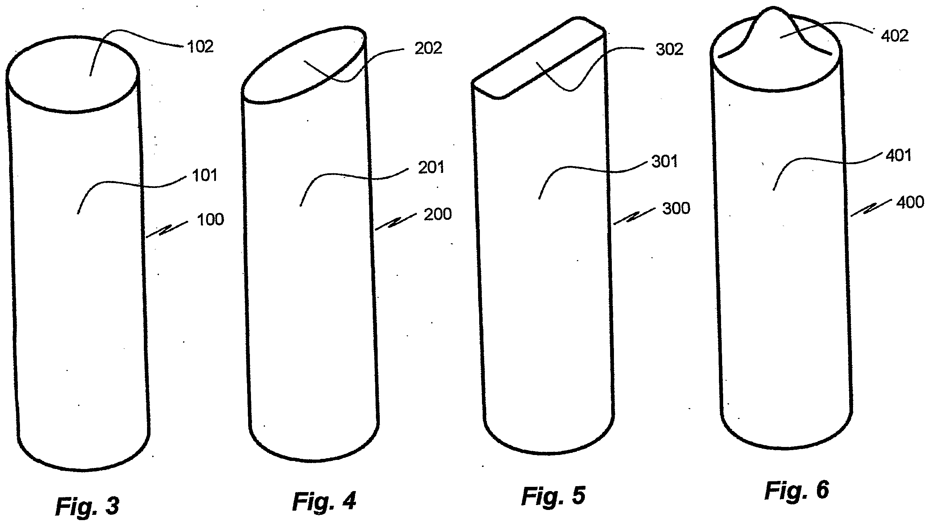

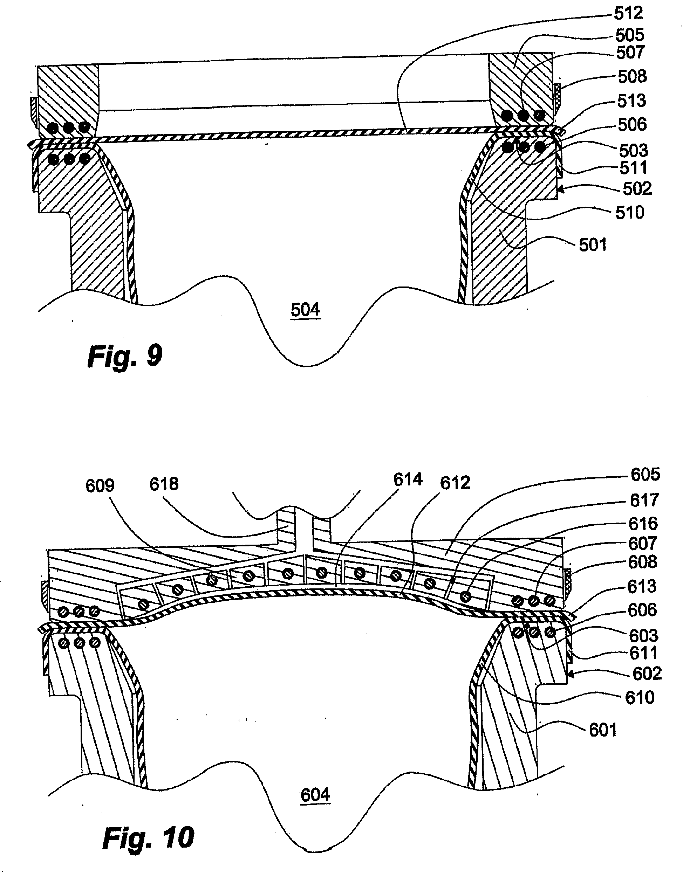

The embodiment 100 of the ultrasound probe cover of the invention illustrated in Fig. 3 comprises a tube member 101 of extruded polyurethane having a wall thickness of about 40 µm and a flat top member 102 of polyurethane sheet material of same thickness joined to and closing one (distal) end of the tube member 101. Over a circular flange 103 the top member 102 is integral with the tube member 101 (Fig. 7). The integral joint between the members 101, 102 is produced in the manner illustrated in Fig. 9. To avoid contact of the flange 103 and sensitive tissue, the ultrasound probe cover 100 is turned inside out (Fig. 8) prior to use to assume the configuration shown in Figs. 3 and 8. The top member 102 of the ultrasound probe cover 100 is circular and of the same diameter as the tube member 101.

Instead of a circular top member 102, top members of other form can be used, such as an elliptic top member 202 (Fig. 4) joined to a cylindrical tube member 201 forming a probe cover 200 or a rectangular top member 302 with rounded corners (Fig. 5) joined to a cylindrical tube member 301 forming a probe cover 300. It is also possible to use top members that are not flat such as, for instance, a dome shaped top member 402 of the embodiment of Fig. 6 joined to the cylindrical tube member 401 forming a probe cover 400. The dome shaped top member 402 or other non-flat top members can be produced, for instance, from a sheet of polymer material by heat forming, in particular by vacuum heat forming.

The integral joint between the tube element and the top element of the probe cover of the invention can be produced in the manner shown in Figs. 9 and 10. A heat welding apparatus comprises a cylindrical aluminum alloy welding bolster 501 having an outer cylindrical face 502, a flat annular top face 503 and a central bore 504 that narrows in a direction away from top face 503. An extruded polyurethane tube 510 is disposed in the bore 504 of the welding bolster 501 and mounted by folding its one end section 511 so as to overlap the flat top face 503 and an adjoining portion of the cylindrical external face 502. To facilitate folding the diameter of the welding bolster 501 at the cylindrical external face 502 is slightly smaller than the inner diameter of the polymer tube 510. A circular sheet 512 of same polymer material as the tube member 510 of larger diameter than the diameter of the welding bolster 501 is placed on top of the tube member 510. The sheet 512 is secured in this position by posing an annular holder 505 of same material as the welding bolster 501 axially aligned with the axis of the welding bolster 501 on the sheet 512. The welding bolster 501 and/or the holder 505 is equipped with insulated resistive heating wires 506, 507 disposed below or above, respectively, of the annular top face 503 or a corresponding flat face on the holder 505. By these means the flat faces of the bolster 501 and/or the holder 505 are heated to a fusing temperature of the polymer material of the tube 510 and the sheet 511 for a time sufficient to substantially soften or melt the clamped portions of the tube 510 and the sheet 512, which are thereby bonded to each other. Portions 511, 513 of the tube 510 and the sheet 512, respectively, disposed externally of the face 503 are cut off by the downward movement of an annular cutter 508. After lifting the holder 505, the thus produced probe head cover 100 is removed from the apparatus. After turning the probe cover inside out, possibly sterilizing it and/or filling it with a measured amount of ultrasound gel, it is packed, and the package is sealed. The probe head cover of the invention can be packed in any desired manner, for instance in a telescopically folded configuration. Ultrasound probe covers with differently shaped top members, such as those illustrated in Figs. 3 to 6, can be manufactured by correspondingly formed combinations of welding bolsters and holders. The probe head cover 400 of Fig. 6 comprising a domed top member 402 can be manufactured by a similar welding bolster 601 in combination with a welding holder 605 having a negative dome shaped central section 609 provided with electrical resistive heating elements 616 and bores 617 in communication with a vacuum source (not shown) by means of a conduit 618. By applying vacuum and heat to the void 614, a central portion of the originally flat sheet 612 is softened and drawn in the direction of the domed shaped section 609 of the holder 602. After cooling under vacuum, the central portion of the sheet 612 has assumed a permanent domed shape. Elements in Fig. 10 nos. 602, 603, 604, 606, 607, 608, 610, 611, 613 are functionally equivalent to elements 502, 503, 504, 506, 507, 508, 510, 511, 512, respectively, of the apparatus of Fig. 9.

The tube and top members of the invention can also be connected by means of an adhesive. For this purpose apparatus similar to those of Figs. 9 and 10 can be used, in which heating elements 503, 507 and 603, 607, respectively, have been omitted. After mounting the tube member 510, 610 on the bolster 501 or 601, respectively, an adhesive is applied on the free face of the tube member 510; 610 facing away from the face 503; 603 of the bolster 501; 601, whereupon the sheet 512; 612 is placed on and pressed against the adhesive bearing free face of the sheet 510; 610. The connection is finished by allowing the solvent to evaporate. Adhesives suitable for polyurethane probe covers comprise solutions of polyurethane, such as hydroxyl terminated polyurethane, in an organic solvent such as acetone or methyl ethyl ketone. Other kinds of adhesives, such as reactive polyurethane adhesives, can also be used.

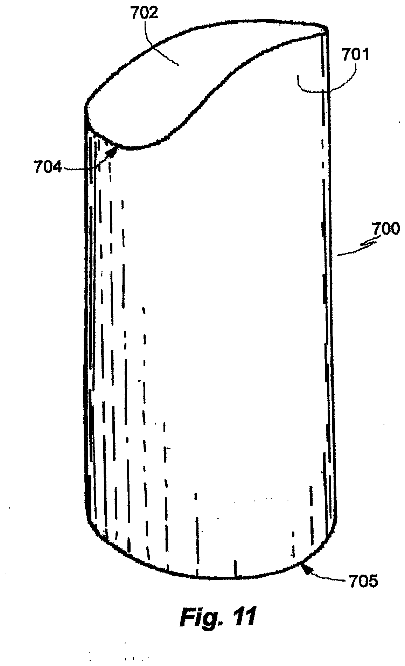

The embodiment of the probe cover 700 of the invention illustrated in Fig. 11 differs from the other embodiments in that the rectangular top member 702 with rounded short sides is attached to a tube member 701 of which the top edge 704 does not extend perpendicularly to the tube member axis, that is, in one plane. Since the top edge 704 is wavy, the top member 702 is not flat but bent, the distance of its short sides to the proximal edge 705 of the tube member 701 being shorter than of its central portion. The probe cover 700 can be manufactured, for instance, by a welding bolster/holder apparatus with saddle formed contact faces corresponding to the annular top face 503 or the corresponding annular face on the holder 505, respectively, of the welding apparatus of Fig 9.

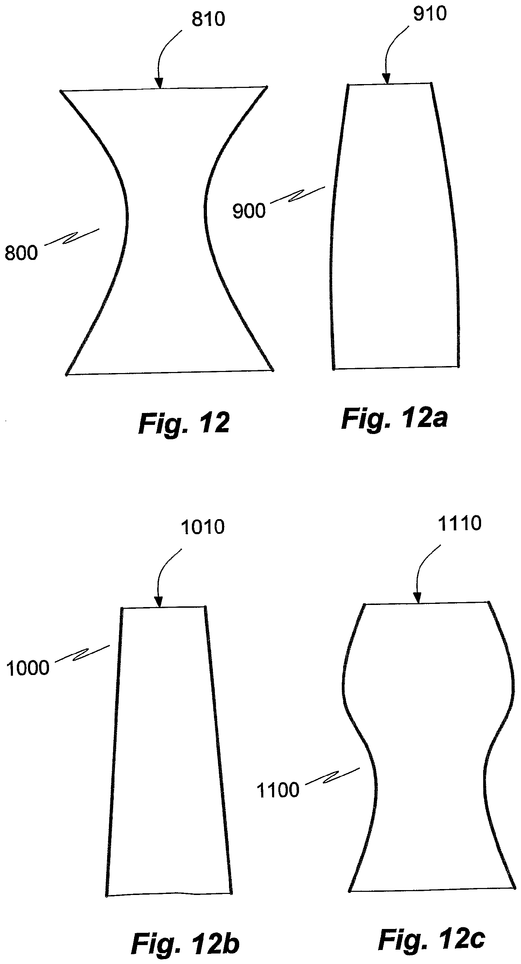

Figs. 12 - 12c illustrate exemplary non-cylindrical tube members 800, 900, 100, 1100 of the invention made by thermoforming of cylindrical tube blanks. Reference nos. 810, 910, 1010, 1110 indicate the front ends of the members 800, 900, 100, 1100, respectively at which top members of desired shape can be mounted by welding or by use of an adhesive. The tube members 800 and 1200 comprise a throat. The lumen of the tube members 900 (frustrum of a cone) and 1000 is narrowing in the direction of their front ends 910, 1010.

The device and method of the invention will be understood from the foregoing description, and it is apparent that various changes may be made in the form, construct steps, and arrangements of the parts and steps thereof. The forms herein described are merely preferred exemplary embodiments thereof.

Although the present invention has been described in relation to particular embodiments thereof, many other variations and modifications and other uses will become apparent to those skilled in the art. It is preferred, therefore, that the present invention be limited not by the specific disclosure herein, but only by the appended claims.

## Claims

1. Ultrasound probe cover comprising a flexible polymer tube member having an open first end and a second end; a seamless top member closing the open first end, and wherein the originally separate tube and top members are joined to each other adhesively by welding or by use of an adhesive forming an outwardly extending flange that is turned inside the probe cover in the final configuration.

2. The cover of claim 1, wherein the tube member and the top member are of the same material.

3. The cover of claim 1 or 2, wherein the tube member has been cut to size from a tube of polymer material produced by extrusion.

4. The cover of any of claims 1 to 3, wherein the top member is of circular or elliptic form or of a rectangular form with rounded corners.

5. The cover of any of claims 1 to 3, wherein the top member is substantially flat.

6. The cover of any of claims 1 to 3, wherein the top member is dome shaped.

7. The cover of any of claims 1 to 6, substantially consisting of said tube member and said top member.

8. The cover of any of claims 1 to 7, wherein said cover is comprised of polyethylene, polypropylene or polyurethane.

9. The cover of any of claims 1 to 8 having a wall with a thickness of from 4 µm to 200 µm.

10. The cover of any of claims 1 to 8 having a wall thickness of from 20 µm to 100 µm.

11. The cover of any of claims 1 to 8 having a wall thickness of about 40 µm.

12. The cover of any of claims 1 to 11, wherein the cover has a lumen and further comprising ultrasound gel disposed in the lumen adjacent to the top member.

13. The cover of claim 12, wherein the cover has a transverse wall or fold proximally of the ultrasound gel.

14. A process of making an ultrasound probe cover comprising the steps of:
**(A)** forming a flexible polymer tube member;
**(B)** passing the tube member through an annular heat welding support;
**(C)** folding one end of the tube member over the welding support over an angle of 90° or more;
**(D)** positioning a sheet of seamless polymer foil as top member on a folded portion of the tube member, clamping annular abutting portions of the tube member and the polymer foil against each other;
**(E)** heating the clamped portions to a temperature near the melting point of the polymer for a time sufficient to make them integrate;
**(F)** optionally cooling the integrated portions to a temperature below the melting point of the polymer;
**(G)** optionally cutting off polymer material extending radially outwardly of the support;
**(H)** stopping the clamping;
**(I)** removing the probe cover from the support;
**(K)** turning the probe cover inside out to obtain the final configuration.

15. The process of claim 14, further comprising a step of heat forming that is operable to produce a heat shaped top member, in particular a dome shaped top member.

## Patentansprüche

1. Ultraschallsondenschutzhülle, aufweisend ein biegsames Polymerröhrenelement mit einem offenen ersten Ende und einem zweiten Ende; ein nahtloses oberes Element, das das offene erste Ende schließt, und
wobei die ursprünglich getrennten Röhren- und oberen Elemente durch Schweißen oder unter Verwendung eines Klebstoffs haftend miteinander verbunden sind, wodurch ein sich nach außen erstreckender Flansch ausgebildet wird, der in der endgültigen Konfiguration in die Sondenschutzhülle gestülpt wird.

2. Schutzhülle nach Anspruch 1, wobei das Röhrenelement und das obere Element aus dem gleichen Material hergestellt sind.

3. Schutzhülle nach Anspruch 1 oder 2, wobei das Röhrenelement aus einer Röhre aus durch Extrudieren hergestelltem Polymermaterial zugeschnitten worden ist.

4. Schutzhülle nach einem der Ansprüche 1 bis 3, wobei das obere Element eine kreisförmige oder elliptische Form oder eine rechteckige Form mit gerundeten Ecken hat.

5. Schutzhülle nach einem der Ansprüche 1 bis 3, wobei das obere Element im Wesentlichen flach ist.

6. Schutzhülle nach einem der Ansprüche 1 bis 3, wobei das obere Element kuppelförmig ist.

7. Schutzhülle nach einem der Ansprüche 1 bis 6, die im Wesentlichen aus dem Röhrenelement und dem oberen Element besteht.

8. Schutzhülle nach einem der Ansprüche 1 bis 7, wobei die Schutzhülle aus Polyethylen, Polypropylen oder Polyurethan gebildet ist.

9. Schutzhülle nach einem der Ansprüche 1 bis 8, die eine Wand mit einer Dicke von 4 µm bis 200 µm aufweist.

10. Schutzhülle nach einem der Ansprüche 1 bis 8, die eine Wanddicke von 20 µm bis 100 µm besitzt.

11. Schutzhülle nach einem der Ansprüche 1 bis 8, die eine Wanddicke von etwa 40 µm hat.

12. Schutzhülle nach einem der Ansprüche 1 bis 11, wobei die Schutzhülle ein Lumen besitzt und ferner Ultraschallgel aufweist, das im Lumen neben dem oberen Element angeordnet ist.

13. Schutzhülle nach Anspruch 12, wobei die Schutzhülle eine Querwand oder Falte proximal des Ultraschallgels hat.

14. Verfahren zum Herstellen einer Ultraschallsondenschutzhülle, umfassend die Schritte:
(A) Ausbilden eines biegsamen Polymerröhrenelements;
(B) Leiten des Röhrenelements durch einen ringförmigen Wärmeschweißträger;
(C) Falten eines Endes des Röhrenelements über den Schweißträger über einen Winkel von 90° oder mehr;
(D) Positionieren einer Schicht aus nahtloser Polymerfolie als oberes Element auf einen gefalteten Abschnitt des Röhrenelements, wobei ringförmige aneinandergrenzende Abschnitte des Röhrenelements und der Polymerfolie gegeneinander geklemmt werden;
(E) Erwärmen der geklemmten Abschnitte auf eine Temperatur nahe dem Schmelzpunkt des Polymers eine Zeit lang, die ausreicht, dass sie sich integrieren;
(F) optional Abkühlen der integrierten Abschnitte auf eine Temperatur unter dem Schmelzpunkt des Polymers;
(G) optional Abschneiden des Polymermaterials, das sich radial nach außen des Trägers erstreckt;
(H) Abbrechen des Klemmens;
(I) Entfernen der Sondenschutzhülle von dem Träger;
(K) Umstülpen der Sondenschutzhülle, um die endgültige Konfiguration zu erhalten.

15. Verfahren nach Anspruch 14, welches ferner einen Schritt zum Warmformen umfasst, der funktionsfähig ist, ein warm ausgeformtes oberes Element, insbesondere ein kuppelförmiges oberes Element herzustellen.

## Revendications

1. Capuchon de sonde à ultrason comprenant un élément de tube polymère souple ayant une première extrémité ouverte et une seconde extrémité ; un élément supérieur sans soudure fermant la première extrémité ouverte, et
dans lequel le tube et des éléments supérieurs séparés à l'origine sont assemblés les uns aux autres de manière adhésive par soudage ou au moyen d'un adhésif formant une bride s'étendant vers l'extérieur qui est tournée à l'intérieur du capuchon de sonde dans la configuration finale.

2. Capuchon selon la revendication 1, dans lequel l'élément de tube et l'élément supérieur sont du même matériau.

3. Capuchon selon la revendication 1 ou 2, dans lequel l'élément de tube a été découpé aux dimensions à partir d'un tube de matériau polymère produit par extrusion.

4. Capuchon selon l'une quelconque des revendications 1 à 3, dans lequel l'élément supérieur est de forme circulaire ou elliptique ou d'une forme rectangulaire avec des coins arrondis.

5. Capuchon selon l'une quelconque des revendications 1 à 3, dans lequel l'élément supérieur est sensiblement plat.

6. Capuchon selon l'une quelconque des revendications 1 à 3, dans lequel l'élément supérieur est en forme de dôme.

7. Capuchon selon l'une quelconque des revendications 1 à 6, sensiblement constitué dudit élément de tube et dudit élément supérieur.

8. Capuchon selon l'une quelconque des revendications 1 à 7, dans lequel ledit capuchon est composé de polyéthylène, de polypropylène ou de polyuréthane.

9. Capuchon selon l'une quelconque des revendications 1 à 8 ayant une paroi dotée d'une épaisseur de 4 µm à 200 µm.

10. Capuchon selon l'une quelconque des revendications 1 à 8 ayant une épaisseur de paroi de 20 µm à 100 µm.

11. Capuchon selon l'une quelconque des revendications 1 à 8 ayant une épaisseur de paroi d'environ 40 µm.

12. Capuchon selon l'une quelconque des revendications 1 à 11, dans lequel le capuchon a une lumière et comprend en outre un gel à ultrason disposé dans la lumière adjacente à l'élément supérieur.

13. Capuchon selon la revendication 12, dans lequel le capuchon a une paroi ou un pli transversal(e) proximal(e) au gel à ultrason.

14. Procédé de fabrication d'un capuchon de sonde à ultrason comprenant les étapes de :
(A) formation d'un élément de tube polymère souple ;
(B) passage de l'élément de tube à travers un support de soudage thermique annulaire ;
(C) pliage d'une extrémité de l'élément de tube sur le support de soudage sur un angle de 90° ou plus ;
(D) positionnement d'une feuille de film polymère sans soudure comme élément supérieur sur une partie pliée de l'élément de tube, serrant des parties de butée annulaires de l'élément de tube et du film polymère l'une contre l'autre ;
(E) chauffage des parties serrées à une température proche du point de fusion du polymère pendant une durée suffisante pour les faire s'intégrer ;
(F) refroidissement éventuel des parties intégrées à une température sous le point de fusion du polymère ;
(G) découpe éventuelle d'un matériau polymère s'étendant radialement vers l'extérieur du support ;
(H) stoppage du serrage ;
(I) retrait du capuchon de sonde du support ;
(K) rotation du capuchon de sonde à l'envers pour obtenir la configuration finale.

15. Procédé selon la revendication 14, comprenant en outre une étape de formation thermique qui permet de produire un élément supérieur formé par la chaleur, en particulier un élément supérieur en forme de dôme.
